# EUROPEAN PATENT SPECIFICATION

(11) **EP 4 251 104 B1**
(45) Date of publication and mention of the grant of the patent: **14.10.2026**
(21) Application number: 21740436.7
(22) Date of filing: 29.06.2021
(51) Int. Cl.: B05B 7/04, B05B 11/10

(54) **A DEVICE FOR THE DELIVERY OF A MIST OF A DOSE OF AN OPHTHALMIC LIQUID**
VORRICHTUNG ZUR ABGABE EINES NEBELS EINER DOSIS EINER OPHTHALMISCHEN FLÜSSIGKEIT
DISPOSITIF DE DISTRIBUTION D'UN BROUILLARD D'UNE DOSE D'UN LIQUIDE OPHTALMIQUE

(30) Priority: 30.11.2020 DK PA202070800
(43) Date of publication of application: 04.10.2023
(73) Proprietor: Eye-Go A/S, 2970 Hørsholm (DK)
(72) Inventor: NAGEL, Henrik, 2800 Kgs. Lyngby (DK); WESTERLUND, Joel Vion, 24231 Hörby (SE); GYRN, Steffen, 4100 Ringsted (DK)
(74) Representative: Zacco Denmark A/S
(86) International application number: PCT/EP2021/067824
(87) International publication number: WO 2022/111865

(56) References cited:
- WO-A2-2005/048875
- US-A- 5 881 956
- US-A1- 2018 235 807

## Description

The present invention relates to an improved device for the delivery of a mist of a dose of an ophthalmic liquid, such as an ophthalmic medicament or a saline solution, of the type including a housing with a holding chamber having a discharge opening, for holding the single dose of liquid to be delivered, a pump for pumping, via a dose supply conduit, the dose of the liquid to the holding chamber, the pump being connected with a container comprising a neck and containing a plurality of the doses, and an air flow conduit for supplying a flow of air to the holding chamber so as to force the dose delivered by the pump to the holding chamber out through the discharge opening.

Documents US 5,881,956, WO15/114,139 and WO17/21,168 disclose devices of the aforementioned type.

There is a need for such a device that allows for one or more of the following: pumping of very small amounts of an ophthalmic liquid, such as an ophthalmic medicament or saline solution, corresponding to the aforementioned single dose which may by way of example be in the order of 6 microliter, a convenient and quick priming of the pump, as well as allowing the pump to be made with small dimensions so it may be placed within a relatively small size device housing.

The invention of claim 1 solves this need, wherein the ophthalmic liquid is delivered to the user via a discharge opening of the device.

The ophthalmic liquid may be delivered to the user in a form mixed in a mixing chamber with air.

Many components of the pump may conveniently be formed by plastic injection molding.

Preferred embodiments are defined in the dependent claims.

### Brief description of the drawings

Figs. 1a and 1b are perspective views in different configurations of a device of the present invention,
Figs. 2a and 2b are partial sectional views of the device of figs. 1a and 1b in the two respective configurations,
Fig. 3a is a perspective view of a pump and a base plate on which it is mounted in the device of the invention,
Figs. 3b and 3c are cross-sectional views of the pump, mounted on the base plate of the device shown in fig. 3a, in different positions during pumping,
Figs. 4a and 4b is a perspective, partial cross-sectional and full cross-sectional view, respectively, showing the pump mounted to the base plate,
Figs. 5a and 5b are views similar to those of figs. 3b and 3c, respectively, and showing the assembled pump,
Figs. 6a and 6b are exploded views of the base plate, seen from below and above, respectively, and
Figs. 7a-7f are cross-sectional view similar to those of fig. 3b and 3c, illustrating liquid flow through the pump during priming.

### Detailed description

The invention will now be explained in more details by reference to an embodiment.

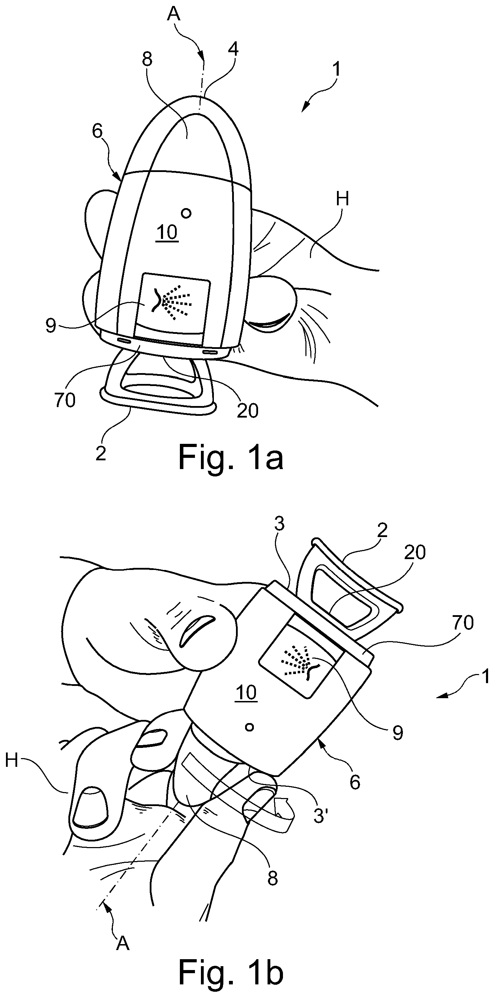

Figs. 1a-1b show perspective views in different configurations of a device 1 of the present invention, for the consecutive delivery of single doses of an ophthalmic liquid, each dose to be carried to a user's eye as small droplets in a stream of carrier air. As shown, the device 1 is sized to be held in a hand H of the user.

The device 1 includes a housing 6 with a front wall 10 and an opposite rear wall 15, and has a drive for preparing the device 1 for each delivery of a single dose by the user rotating with his hand H a handle/head 8 of the device 1 about an axis A, as seen in fig. 1b. The head 8 is rotatably mounted to a rear end 3' of the housing 6 and defines the rear end 4 of the device 1. A release button 9 in the front wall 10 allows the user to bring about the delivery of each single dose in a respective stream of carrier air via a discharge opening 20 formed in a base plate 70 that defines the front end 3 of the housing 6. The discharge opening 20 may be formed as a nozzle having atomizing capabilities.

As shown, an abutment structure 2 connected to the base plate 70 allows the user to position and hold the device 1 against the area surrounding his eye before pressing the release button 9.

The shown device 1 has some features in common with the device disclosed in documents WO15/114,139 and WO17/21,168, in particular in having inside the housing 6 a liquid ophthalmic medicament/saline solution container/cartridge connected to a pump by which each single dose of the ophthalmic liquid is driven out from the cartridge into a single dose holding chamber. The single dose then sits ready in the single dose holding chamber, awaiting a first stream of air that presses the single dose out of the single dose chamber into a mixing chamber arranged immediately downstream of the single dose chamber. The single dose thus entering the mixing chamber is mixed with a second stream of air simultaneously entering the mixing chamber, and a combined flow of the first and second air streams together with the single dose of the ophthalmic liquid then exits the mixing chamber at the discharge opening 20. The dose of the ophthalmic liquid is thereby delivered to the user as a mist, at a speed determined i.a. by the pressure of the flow of air.

The present invention is particularly suitable where the volume of the dose of the ophthalmic liquid required to be dispensed for the treatment of a person's eye is very low, such in the order of 5-30 microliters, such as around 6 microliters, and the volume of the dose holding chamber is preferably the same, or essentially the same, as the volume of the single dose. In conventional ophthalmic liquid dispensers one or more drops are typically dispensed, having each a volume in the order of more than 30 microliters, or even more than 60 microliters, leading to an excessive dispensing.

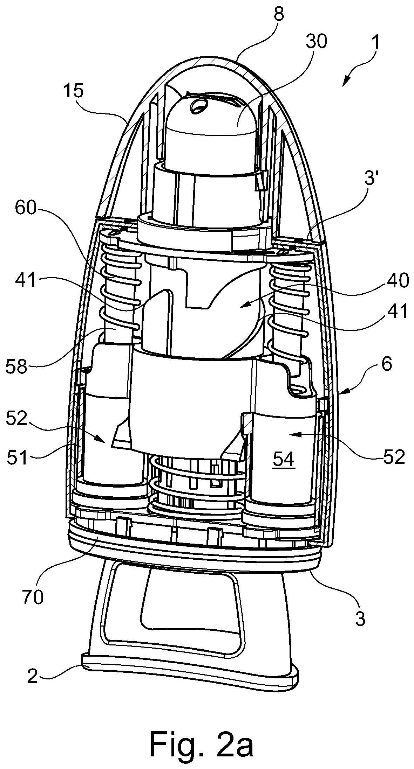

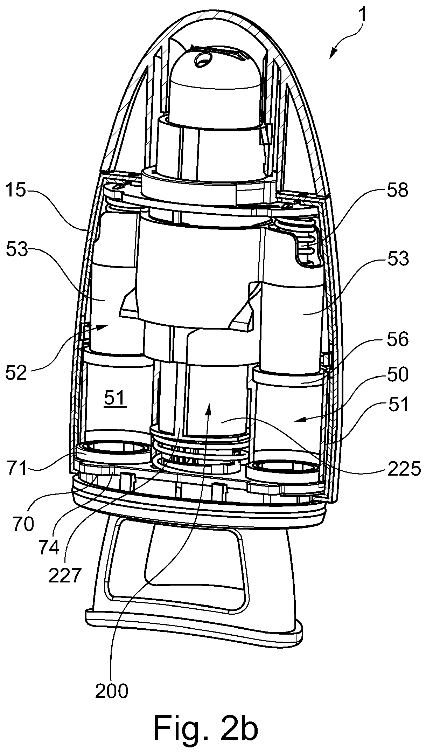

Figs. 2a and 2b are partial sectional views of the device 1 in two respective configurations before and after a 180° rotation of the head 8, allowing inspection of some of the components inside the housing 6, including the top portion of the aforementioned container/cartridge, referenced by numeral 30, as well as two pistons 52 that are arranged to move back and forth (in the drawings shown as up and down) parallel with the axis A, inside a respective air chamber 50 which is closed at one end by the aforementioned base plate 70. The two air chambers 50 are arranged essentially symmetrically about the cartridge 30 and about the axis A.

In the context of the present invention the cartridge 30 is preferably a collapsible container, open only at a discharge opening, and configured with a flexible side wall such that it will collapse as the ophthalmic liquid is drawn from the cartridge 30, without drawing air into the cartridge 30. Alternatively, the cartridge 30 may have a displaceable wall portion such that the internal volume of the cartridge 30 is reduced as liquid is drawn out, without drawing air into the cartridge 30. The cartridge 30 may, by way of example, contain 10-1000 doses of the ophthalmic liquid.

Each of the aforementioned air chambers 50 is defined in part by a cylindrical wall 51, shown in cross-section in figs. 2a and 2b, sealed to an annular structure 71 on the base plate 70. The pistons 52 each have a piston head 56 delimiting together with a portion of the base plate 70 and one of the cylindrical wall 51 the inside of a respective air chamber 50. Fig. 2b shows the two pistons 52 having been moved to their upper, withdrawn position by the user having rotated the head 8. For this movement, the head 8 includes a hollow cylindrical shaft 40 rotatably received within the housing 6 through an opening in the rear end 3' and enclosing the container 30. The shaft 40, which is a part of the aforementioned drive, includes on its outer face a pair of opposite, helically running ridges 41 defining cams acting on a corresponding cam follower (not shown) arranged each one of a respective non-rotatable piston rod 53 connected to each piston head 56. The two pistons 52 are temporarily held in their fully withdrawn position shown in fig. 2b by respective tabs (not shown) releasably engaging in this position the piston rods 53, and configured to be disengaged from the piston rods 53 when the user presses the button 9.

As will be understood, the upward movement of the pistons 52 is brought about by rotating the shaft 40 about the axis A whereby the cam followers will ride on the ridges 41. By this upward movement of the pistons 52, air is simultaneously drawn into the two air chambers 50 via respective apertures 74 that are formed in the base plate 70. The apertures 74 communicate with the outside of the device 1 and include each a non-return valve (not shown).

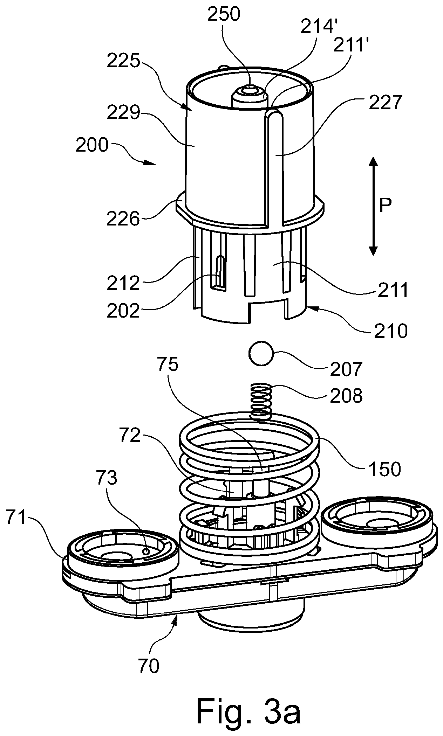

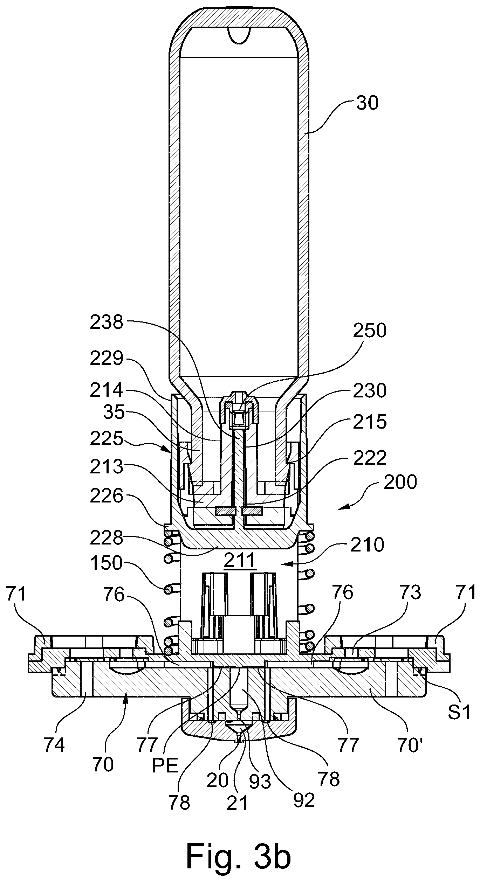

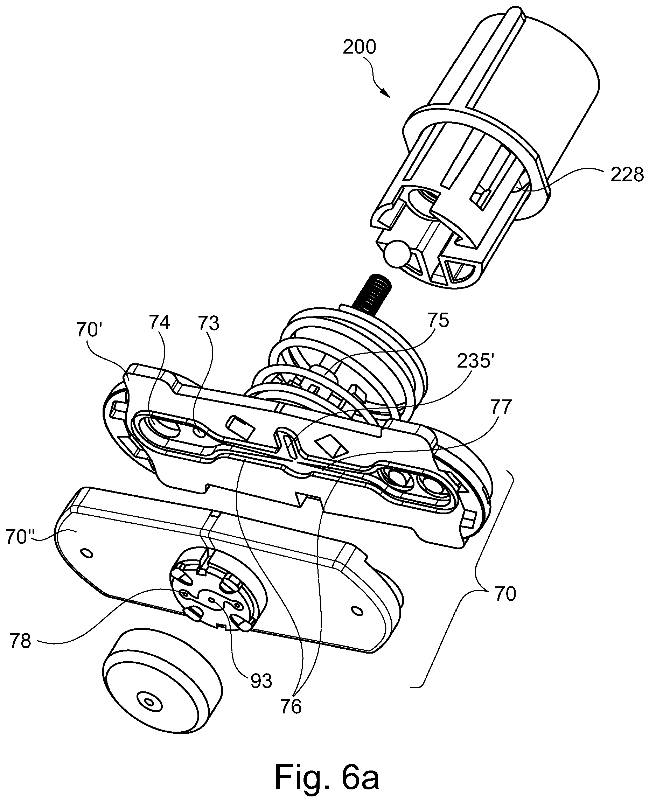

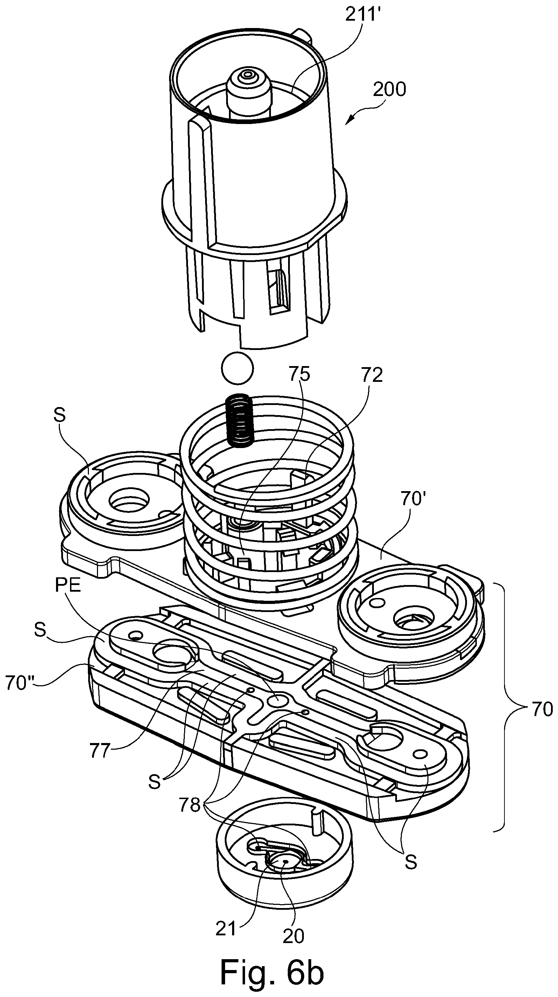

A respective spring 58 positioned between the pistons 52 and the rear end 3' of the housing 6 drives the pistons 52 back to their lower position shown in fig. 2a when the user presses the button 9 to release the pistons 52 from their engagement with aforementioned tabs, at the same time expelling air from the air chambers 50 via an aperture 73 formed in the base plate 70. Air exiting in this manner the air chambers 50 flows towards and into the single dose holding chamber 92 via air flow conduits 76, 77 formed in the base plate 70 at the bottom of the air chambers 50, see fig. 3a and 3b. Further details of the base plate 70 are shown in figs. 6a and 6b from which it appears how the base plate 70 preferably may be formed by tightly assembling two plates 70', 70" having recesses defining the aforementioned flow conduits 73, 76, 77, as well as segments 235' of further conduits and required valves. An elastomeric material sealing part which may be co-molded with one of the plates 70" and which is composed of a plurality of preferably interconnected strings S, as shown in fig. 6b, cooperates with corresponding ridges S1, of which one is shown in fig. 3b, formed on the other one of the plates 70".

Preferably, as in the prior art referred to above, the air expelled from each air chambers 50 defines a first and a second stream of air, serving the aforementioned purpose, and flowing through respective air flow conduits 77, 78 that may be branched off from a primary conduit 76 extending from each air chamber 50, one, first flow conduit 77 for the first stream of air connecting with the dose holding chamber 92 and another, second conduit 78 connecting with the mixing chamber 21 for the second stream of air. The various air flow conduits 76, 77, 78 preferably are formed as recesses in one of the plates 70", as seen in fig. 6a, and the aforementioned ridges S1 may be formed on that same plate 70" along the length of the various air flow conduits 76, 77, 78 so as to seal the air flow conduits 76, 77, 78 by being pressed into corresponding strings S of the sealing material.

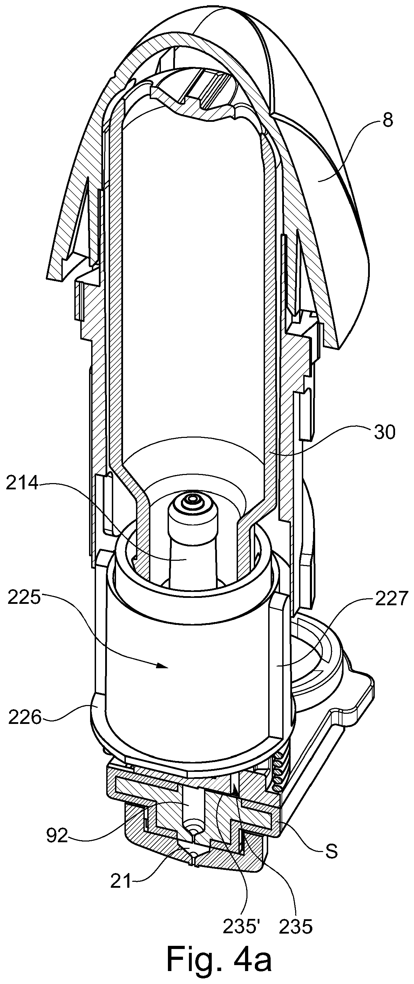

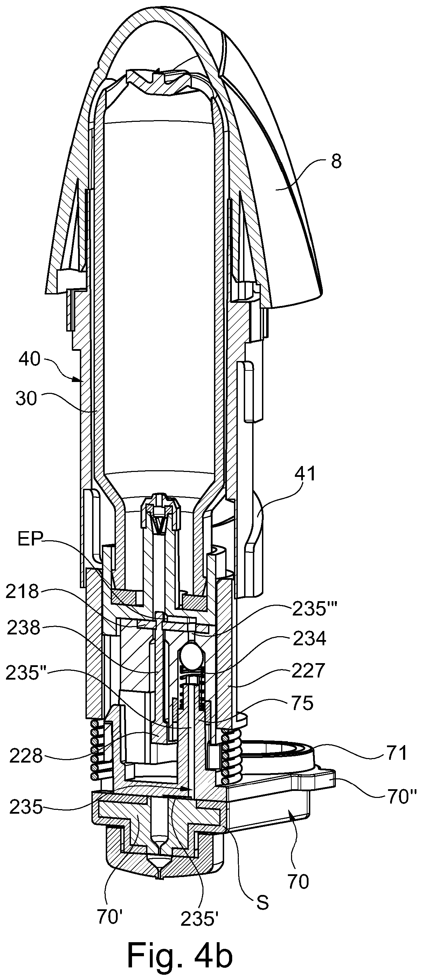

As shown, the dose holding chamber 92 has a discharge opening 93 communicating with the mixing chamber 21, whereby the first stream of air may force liquid contained in the dose holding chamber 92 into the mixing chamber 21 via the discharge opening 93, which is located at a distal end of the dose holding chamber 92 that is opposite a proximal end PE thereof where the first flow conduit opens up into the holding chamber 92. As seen best in fig. 4b, a dose supply conduit 235 for supplying doses of the ophthalmic liquid to the dose holding chamber 92 has a segment 235' that also opens up at the proximal end PE of the dose holding chamber 92.

The shown device 1 differs from the prior art device disclosed in documents WO 15/114,139 and WO 17/21,168 in *inter alia* having a specially configured pump 200 according to the invention. The drive that includes the shaft 40 also brings about a stroke of the pump 200, to drive a single dose of the ophthalmic liquid from the container 30 into the single dose chamber 92 via the dose supply conduit as the shaft 40 is rotated. In this manner, rotation of the head 8 for one draws air into the air chambers 50 and secondly also pumps a single dose of the ophthalmic liquid from the container 30 into the holding chamber 92, whereby the subsequent pressing of the button 9 will deliver this single dose to the user via the discharge opening 20 by the combined streams of air, that are discharged from the air chambers 50 by the action of the respective springs 58, acting as a carrier.

As explained below, the specially configured pump 200 of the device of the present invention i.a. allows for pumping very small amounts of the medicament, namely corresponding to the aforementioned single dose which may by way of example be 6 microliter, for a convenient and quick priming of the pump 200, as well as for allowing the pump 200 to be made with small dimensions so it may be placed within the relatively small size housing 8 of the device 1.

Turning now to fig. 3a, shown is a pump 200 of the device of the invention, having been previously assembled and in the process of being mounted to the base plate 70 of the device 1. For this, the base plate 70 includes a plurality of upstanding resilient fingers 72 adapted to snap into a corresponding number of recesses 202 formed in a cylindrical wall 211 of a first part 210 of the pump 200, with an upstanding fluid connector 75 mounted to the base plate 70 being configured to be tightly received in a large diameter portion 234 of a dose supply conduit 235, see fig. 4b, that is formed in the first part 210, with the dose supply conduit 235 continuing inside the fluid connector 75. In the dose supply conduit 235 the pump 200 includes a normally closed one-way valve, refer again to fig. 4b, exemplified by the shown ball 207 biased to a normally closed position closing off a segment of the dose supply conduit 235 by a spring 208 held in place by the fluid connector 75. Two opposite guiding slits 212 formed in the cylindrical wall 211 are open at one, lower end and closed at the opposite end.

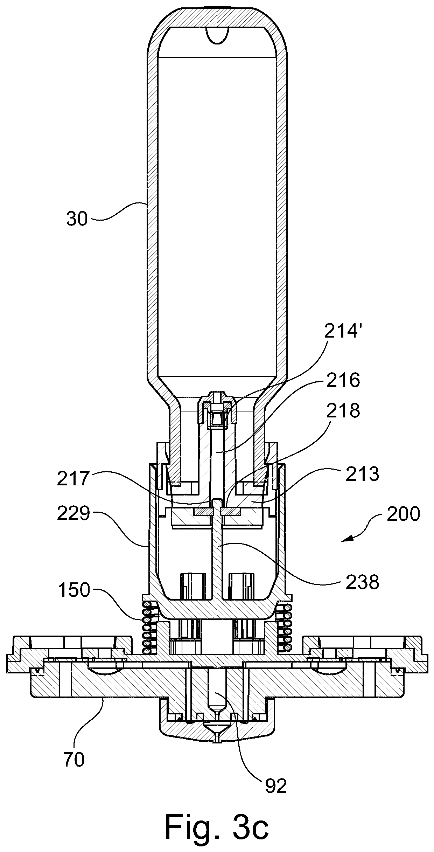

Fig. 3b is a cross-sectional view showing the pump 200 mounted to the base plate 70, with a spring 150 surrounding the first part 210 bearing against the base plate 70 on the one hand and against a peripheral flange 226 of a second part 225 of the pump 200 on the other hand. The second part 225 is displaceable up and down in direction P relative to the first part 210 and, hence, the base plate 70. The second part 225 has a peripheral/cylindrical wall 229 that in a retracted position of the second part 225 surrounds the first part 210; the second part 225 is biased towards an upper, advanced position shown in figs. 3a and 3b by the spring 150. In fig. 3c the second part 225 is shown displaced to the retracted, lower position close to the base plate 70, ready for discharging a single dose of ophthalmic liquid from a pump chamber 216 in the pump 200, via the aforementioned dose supply conduit 235, as explained below.

More specifically, figs. 3b and 3c show the lower, first part 210 as including near the upper end of the cylindrical wall 211 a transverse, annular top wall 213, with an elongated hollow tubular structure 214 extending centrally upwards from the top wall 213. Alongside the tubular structure 214, on an upper extension 211' of the cylindrical wall 211 surrounding a lower portion of the elongated tubular structure 214, is a seat 215 configured for tightly securing the neck 35 of the container 30 to the first part 210, such as by snapping on to a rim of the container 30, with the tubular structure 214 extending inside the neck 35. When secured to the seat 215 the container 30 is held to the first part 210 of the pump 200 in a fixed position relative to the base plate 70.

The tubular structure 214 has a free, distal end 214' remote from the top wall 213 that includes a one-way valve 250, such as a duck bill valve, permitting flow of liquid from the container 30 into the elongated tubular structure 214 via its distal end 214'.

The tubular structure 214 defines a pump chamber 216, best seen in fig. 3c, with the transverse top wall 213 of the first part 210 having a central opening 217 leading in to the pump chamber 216 at a proximal end 214" of the tubular structure 214. An annular seal 218, preferably of a resilient material, such as rubber, shown here as mounted between layers of the top wall 213, surrounds the opening 217.

A lower end of the aforementioned cylindrical wall 229 of the second part 225 of the pump 200 has a bridge 228 that connect to two opposite portions of the flange 226. On assembling the pump 200, the bridge 228 is received at the open ends of the two guiding slits 212 in the first part 210 such that the second part 225 is held against rotation relative to the first part 210 as it moves up and down along direction P, with the bridge 228 moving in a respective one of the two guiding slits 212, up to the closed end of the two slits 212.

An solid elongated pin-shaped pump stem 238, which may have a constant diameter along its length, is connected to the bridge 228 so as to extend into the pump chamber 216 via the opening 217, in sealing engagement with the annular seal 218. The pump stem 238 is arranged to move inside the pump chamber 216 between a retracted position shown in fig. 3c and an advanced position shown in fig. 3b, with a free end 130 of the pump stem 238 being close to the one-way valve 250 in the advanced position.

An elongated, annular fluid flow passage 230 for ophthalmic liquid that is received via the valve 250 and that is displaced from the pump chamber 216 on moving the pump stem 238 to the advanced position, i.e. when the second part 225 of the pump 200 is moving to its advanced position, is defined between the outside of the pump stem 238 and the inside of the tubular structure 214. Where the stem 228 has a constant diameter the annular fluid flow passage 230 preferably has a constant inner and outer diameter; without departing from the invention the stem 228 may have a polygonal cross-section.

Due to the small dimensions, in that the pump chamber 216 is sized to displace on each stroke a volume of the liquid in the order of 6 microliters, sideways support of the stem 238 at the opening 217 is sufficient to steer the stem 228 free from contacting the inside face of the pump chamber 216 as the stem moves inside the pump chamber 216.

As may be understood the spring 150 acts to control movement of the pump stem 238 inside the pump chamber 216, between its retracted and advanced positions. The cylindrical wall 229 of the displaceable second part 225 of the pump 200 has a structure including opposite ribs 227 acting as cam followers engaged in a manner similar for the air pistons 52 with cams (not shown) on the inside of the surrounding hollow shaft 40. In this manner, rotation of the head 8 i) draws liquid into the pump chamber 216 by moving the second part 225 to the position shown in fig. 3c while compressing the spring 150, and then finally ii) allows the spring 150 to drive the second part 225 to its upper position shown in fig. 3b on completion of the rotation where the ribs 227 disengage the cams on the inside of the shaft 40. This latter movement corresponds to a pump stroke, whereby liquid contained in the pump chamber 216 is expelled via the elongated flow passage 230 defined between the pump stem 238 and the inside of the tubular structure 214, to flow into the liquid holding chamber 92 via the dose supply conduit 235.

With the liquid holding chamber 92 now filled the device 1 is ready for medicament delivery by pressing the button 9, leading as explained above, air from the air chambers 50 to purge the liquid holding chamber 92. The liquid holding chamber may then be filled again in the manner explained above, by another rotation of the head 8.

It will be understood that when the liquid is being expelled from the pump chamber 116 the valve 207 opens, and then recloses after completion of the pump stroke by the action of the valve spring 208, eg. to ensure liquid may then be drawn into the pump chamber 216 via the one-way valve 250 only when the pump stem 238 is moved back to its retracted position shown in fig. 3c. Using containers 30 of the collapsible type, such as in the form of a cartridge with an inner, flexible and collapsible bag, allows the cartridge to be emptied without the need for any venting that may compromise sterility. The one-way valve 207 in the dose supply conduit 235 serves in addition the function of preventing upstream contamination in that it acts as a barrier between the liquid in the pump chamber and the surroundings.

Fig. 4a is a perspective, partial cross-sectional view showing the pump mounted to the base plate 70, and fig. 4b is a similar, fully cross-sectional view. The dose supply conduit 235, shown in its full extension in fig. 4b, comprises a first segment 235"' which is formed in the first part 210 of the pump 200 and which has its entry port located in the side of the elongated tubular structure 214 adjacent the opening 217 in the top wall 213, and two further segments 235", 235' formed in the upstanding connector 75 and in the base plate 70, respectively.

As will be understood, the volume of the pump chamber 216 corresponds to the volume of a single dose, which again is related to the volume of the pump stem 238, plus the volume defined by the annular space between the pump stem 238 and the inner face of the elongated tubular structure 214. Typically, with a dose volume of 6 microliters the volume of the pump chamber 216 will be designed to be in the order of 15-20 microliters.

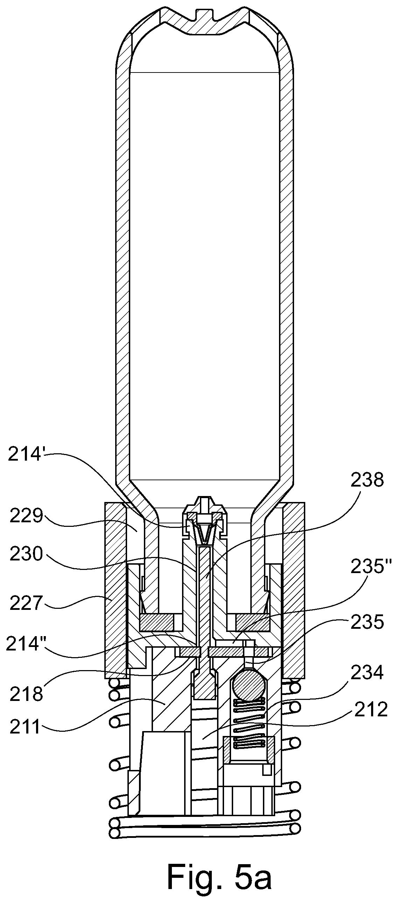

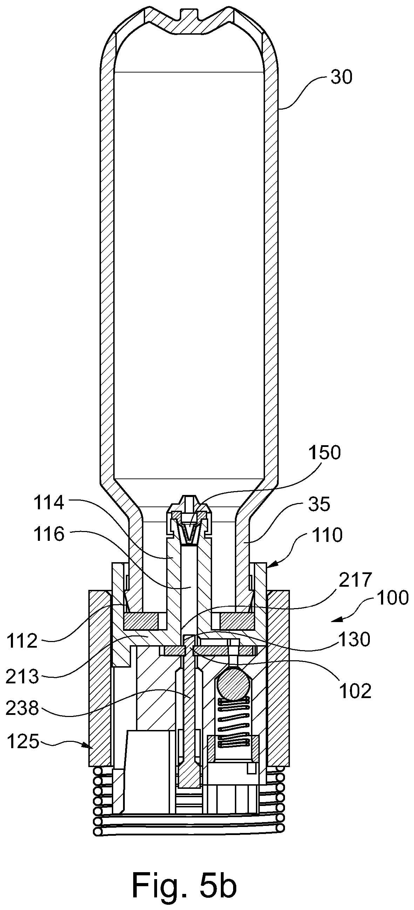

Figs. 5a and 5b are views similar to those of figs. 3b and 3c, respectively, and showing the assembled pump 200, together with the spring 150 compressed or not depending on the position of the second part 225 relative to the first part 210. Pulling the second part 225 away from the first part 210 via the open ends of the slits 212 may be prevented by including a locking structure (not shown).

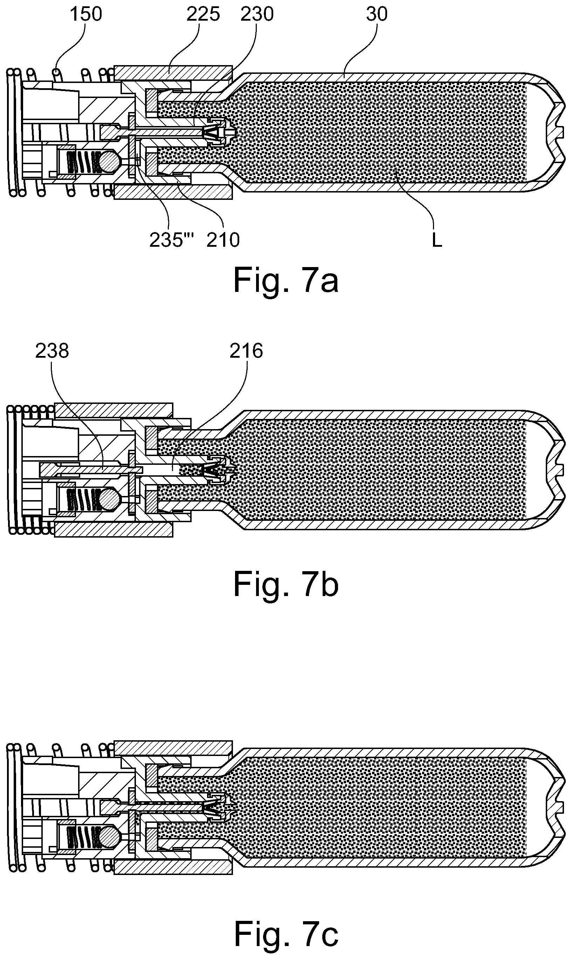

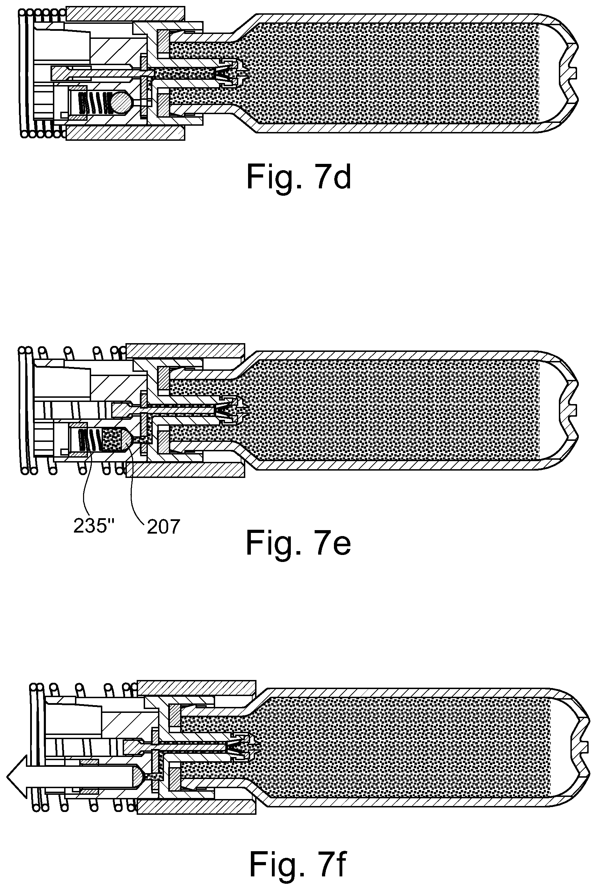

Figs. 7a-7e is a sequence illustrating flow of liquid L, represented by dots, through the pump 200 as the user prepares the device 1 for a first delivery of a dose of the ophthalmic liquid L, i.e. during priming, by rotating the head 8 a prescribed number of times, providing in the shown example a total of four strokes of the pump 200, namely by twice moving the pump stem 238 from its advanced position, which it assumes when the device 1 is delivered to the user, cf. fig. 7a, and by twice moving the pump stem 238 back to its advanced position. During this procedure liquid L is drawn into the pump chamber 216, first to fill the latter; the liquid is then forced into the laterally oriented segment 235'" of the dose supply conduit 235, as shown in fig. 7d, to flow into the segment 235" with the one-way valve 207, thereby eventually opening the one-way valve 207, to then flow into the last segment 235' of the dose supply conduit 235 which opens up into the liquid holding chamber 92 to which the pump 200 may be connected as shown in fig. 4b. As may be understood the volume of the liquid first drawn into the chamber 216 on a first stroke of the stem 238 towards its retracted position, as shown in fig. 7b, will normally correspond to the volume of the stem 238 that it takes up in its fully advanced position inside the chamber 216 and, consequently, to the volume of the liquid holding chamber 92.

## Claims

1. A device (1) for the delivery of a mist of a dose of an ophthalmic liquid, said device (1) comprising a housing (6) with:
- a holding chamber (92) having a discharge opening (93), and being for holding a dose of liquid to be delivered,
- a pump (200) for pumping, via a dose supply conduit (235), said dose of said liquid to said holding chamber (92), said pump (200) being connected with a container (30) comprising a neck (35) and containing a plurality of said doses,
- an air flow conduit (76, 77) for supplying a flow of air to said holding chamber (92) so as to force said dose delivered by said pump (200) to said holding chamber (92) out through said discharge opening (93),
**characterised in** said pump (200) comprising
- a first part (210) with a base (213), an elongated tubular structure (214) extending from said base (213), a seat (215) configured for securing the neck (35) of said container (30) to said first part (210) with said tubular structure (214) extending inside said neck (35), said tubular structure (214) having a distal end including a one-way valve (250) configured to permit flow of said liquid from said container (30) into said elongated tubular structure (214), said tubular structure (214) defining a pump chamber (216), said base (213) having an opening (217) leading in to said pump chamber (216) at a proximal end of said tubular structure (214), a seal (218) surrounding said opening (217), and
- a second part (225) movable relative to said first part (210) and including a pump stem (238) extending into said pump chamber (216) through said opening (217), in sealing engagement with said seal (218), said pump stem (238) moving relative to said first part (210) inside said pump chamber (216) between a retracted position and an advanced position, with a free end (130) of said pump stem (238) closer to said one-way valve (250) in said advanced position, an elongated flow passage (230) for liquid displaced from said pump chamber (216) being defined between said pump stem (238) and the inside of said tubular structure (214) on moving said pump stem (238) to said advanced position,
- said dose supply conduit (235) connecting said elongated flow passage (230) with said liquid holding chamber (92) and including a one-way valve (207, 208) permitting flow of said liquid to said liquid holding chamber (92).

2. The device (1) of claim 1, said second part (225) having a peripheral wall (229) surrounding said first part (210) in said advanced position of said pump stem (238).

3. The device (1) of claim 1 or 2, including a further chamber (21) communicating with said single dose chamber (92) via said discharge opening (93), and a further air flow conduit (78) for supplying a second flow of air to said further chamber (21), said further chamber (21) having a discharge opening (20) for discharge from said device (1) of said forced out single dose together with said second flow of air.

4. The device according to any of the previous claims, further comprising a spring (150) surrounding said first part (210), for controlling said movement of said pump stem (238) inside said pump chamber (216) between said retracted and advanced positions.

5. The device according to any of the previous claims, said dose supply conduit (235) having a liquid entry port (EP) arranged at a proximal end of said tubular structure (214), preferably adjacent said opening (217), a segment (235‴) of said dose supply conduit (235) preferably extending laterally relative to said pump stem (238).

6. The device (1) according to any of the previous claims, wherein said container (70) is collapsible.

7. The device (1) according to any of the previous claims, including an air chamber (58) connected to said air flow conduit (76, 77), a displaceable piston (55) for driving a volume of air out of said air chamber (58), and a drive for controlling displacement of said piston (55).

8. The device (1) according to the previous claim, further comprising a rotatable handle (8), said piston (55) including a piston rod (57) and a piston head (56) received in said air chamber (58), wherein rotation of said handle (8) moves said piston (55) to a retracted position for drawing in said volume of air into said air chamber (58), and wherein said rotation of said handle (8) controls said movement of said second part (225) relative to said first part (210).

9. The device according to any of the previous claims, said first part (210) including said dose supply conduit (235).

## Patentansprüche

1. Vorrichtung (1) zur Abgabe eines Nebels einer Dosis einer ophthalmischen Flüssigkeit, wobei die Vorrichtung (1) ein Gehäuse (6) mit Folgendem umfasst:
- einer Haltekammer (92), die eine Auslassöffnung (93) aufweist und dazu dient, eine Dosis einer abzugebenden Flüssigkeit zu halten,
- einer Pumpe (200) zum Pumpen, über eine Dosiszufuhrleitung (235), der Dosis der Flüssigkeit in die Haltekammer (92), wobei die Pumpe (200) mit einem Behälter (30) verbunden ist, der einen Hals (35) umfasst und eine Vielzahl der Dosen enthält,
- einer Luftstromleitung (76, 77) zum Zuführen eines Stroms von Luft in die Haltekammer (92), um so die Dosis, die durch die Pumpe (200) an die Haltekammer (92) abgegeben wird, durch die Auslassöffnung (93) heraus zu zwingen,
**gekennzeichnet dadurch, dass** die Pumpe (200) Folgendes umfasst:
- einen ersten Teil (210) mit einer Basis (213), einer verlängerten rohrförmigen Struktur (214), die sich von der Basis (213) erstreckt, einem Sitz (215), der zum Sichern des Halses (35) des Behälters (30) an dem ersten Teil (210) mit der rohrförmigen Struktur (214), die sich innerhalb des Halses (35) erstreckt, konfiguriert ist, wobei die rohrförmige Struktur (214) ein distales Ende aufweist, das ein Einwegventil (250) beinhaltet, das dazu konfiguriert ist, einen Strom der Flüssigkeit von dem Behälter (30) in die verlängerte rohrförmige Struktur (214) zu ermöglichen, wobei die rohrförmige Struktur (214) eine Pumpkammer (216) definiert, wobei die Basis (213) eine Öffnung (217) aufweist, die an einem proximalen Ende der rohrförmigen Struktur (214) in die Pumpkammer (216) führt, und einer Dichtung (218), welche die Öffnung (217) umgibt, und
- einen zweiten Teil (225), der relativ zu dem ersten Teil (210) bewegbar ist und einen Pumpenstiel (238) beinhaltet, der sich durch die Öffnung (217) in die Pumpkammer (216) erstreckt, in Dichteingriff mit der Dichtung (218), wobei sich der Pumpenstiel (238) relativ zu dem ersten Teil (210) innerhalb der Pumpkammer (216) zwischen einer zurückgezogenen Position und einer vorgeschobenen Position bewegt, wobei ein freies Ende (130) des Pumpenstiels (238) in der vorgeschobenen Position dichter an dem Einwegventil (250) ist, wobei beim Bewegen des Pumpenstiels (238) in die vorgeschobene Position ein verlängerter Stromdurchgang (230) für Flüssigkeit, die aus der Pumpkammer (216) verschoben wird, zwischen dem Pumpstiel (238) und dem Inneren der rohrförmigen Struktur (214) definiert wird,
- die Dosiszufuhrleitung (235), die den verlängerten Stromdurchgang (230) mit der Flüssigkeitshaltekammer (92) verbindet und ein Einwegventil (207, 208) beinhaltet, das einen Strom der Flüssigkeit in die Flüssigkeitshaltekammer (92) ermöglicht.

2. Vorrichtung (1) nach Anspruch 1, wobei der zweite Teil (225) eine umlaufende Wand (229) aufweist, die den ersten Teil (210) in der vorgeschobenen Position des Pumpenstiels (238) umgibt.

3. Vorrichtung (1) nach Anspruch 1 oder 2, die eine weitere Kammer (21), die mit der Einzeldosenkammer (92) über die Auslassöffnung (93) kommuniziert, und eine weitere Luftstromleitung (78) zum Zuführen eines zweiten Stroms von Luft in die weitere Kammer (21) beinhaltet, wobei die weitere Kammer (21) eine Auslassöffnung (20) zum Auslassen der herausgezwungenen Einzeldosis zusammen mit dem zweiten Strom von Luft aus der Vorrichtung (1) aufweist.

4. Vorrichtung nach einem der vorhergehenden Ansprüche, ferner umfassend eine Feder (150), die den ersten Teil (210) umgibt, zum Steuern der Bewegung des Pumpenstiels (238) innerhalb der Pumpkammer (216) zwischen der zurückgezogenen und vorgeschobenen Position.

5. Vorrichtung nach einem der vorhergehenden Ansprüche, wobei die Dosiszufuhrleitung (235) einen Flüssigkeitseintrittsanschluss (EP) aufweist, der an einem proximalen Ende der rohrförmigen Struktur (214) angeordnet ist, bevorzugt angrenzend an die Öffnung (217), wobei sich ein Segment (235'") der Dosiszufuhrleitung (235) bevorzugt lateral relativ zu dem Pumpenstiel (238) erstreckt.

6. Vorrichtung (1) nach einem der vorhergehenden Ansprüche, wobei der Behälter (70) zusammenschiebbar ist.

7. Vorrichtung (1) nach einem der vorhergehenden Ansprüche, beinhaltend eine Luftkammer (58), die mit der Luftstromleitung (76, 77) verbunden ist, einen verschiebbaren Kolben (55) zum Treiben eines Volumens von Luft aus der Luftkammer (58) und einen Antrieb zum Steuern der Verschiebung des Kolbens (55).

8. Vorrichtung (1) nach dem vorhergehenden Anspruch, umfassend einen drehbaren Griff (8), wobei der Kolben (55) eine Kolbenstange (57) und einen Kolbenboden (56) beinhaltet, der in der Luftkammer (58) aufgenommen ist, wobei eine Rotation des Griffs (8) den Kolben (55) zum Ziehen des Volumens von Luft in die Luftkammer (58) in eine zurückgezogene Position bewegt und wobei die Rotation des Griffs (8) die Bewegung des zweiten Teils (225) relativ zu dem ersten Teil (210) steuert.

9. Vorrichtung nach einem der vorhergehenden Ansprüche, wobei der erste Teil (210) die Dosiszufuhrleitung (235) beinhaltet.

## Revendications

1. Dispositif (1) de distribution d'un brouillard d'une dose d'un liquide ophtalmique, ledit dispositif (1) comprenant un boîtier (6) pourvu de :
- une chambre de retenue (92) présentant une ouverture de décharge (93), et destinée à contenir une dose de liquide à distribuer,
- une pompe (200) pour pomper, par l'intermédiaire d'un conduit d'alimentation en dose (235), ladite dose dudit liquide vers ladite chambre de retenue (92), ladite pompe (200) étant reliée à un contenant (30) comprenant un col (35) et contenant une pluralité desdites doses,
- un conduit d'écoulement d'air (76, 77) pour fournir un écoulement d'air à ladite chambre de retenue (92) de manière à expulser de force ladite dose, délivrée par ladite pompe (200) à ladite chambre de retenue (92), à travers ladite ouverture de décharge (93),
**caractérisé en ce que** ladite pompe (200) comprend :
- une première partie (210) dotée d'une base (213), d'une structure tubulaire allongée (214) s'étendant depuis ladite base (213), d'un siège (215) configuré pour assujettir le col (35) dudit contenant (30) à ladite première partie (210) avec ladite structure tubulaire (214) s'étendant à l'intérieur dudit col (35), ladite structure tubulaire (214) présentant une extrémité distale incluant un clapet anti-retour (250) configuré pour permettre l'écoulement dudit liquide depuis ledit contenant (30) jusque dans ladite structure tubulaire allongée (214), ladite structure tubulaire (214) définissant une chambre de pompe (216), ladite base (213) présentant une ouverture (217) débouchant dans ladite chambre de pompe (216) au niveau d'une extrémité proximale de ladite structure tubulaire (214), un joint d'étanchéité (218) entourant ladite ouverture (217), et
- une seconde partie (225) mobile par rapport à ladite première partie (210) et incluant une tige de pompe (238) s'étendant jusque dans ladite chambre de pompe (216) à travers ladite ouverture (217), en contact étanche avec ledit joint d'étanchéité (218), ladite tige de pompe (238) se déplaçant par rapport à ladite première partie (210) à l'intérieur de ladite chambre de pompe (216) entre une position rétractée et une position avancée, une extrémité libre (130) de ladite tige de pompe (238) étant plus proche dudit clapet anti-retour (250) dans ladite position avancée, un passage d'écoulement allongé (230) pour le liquide déplacé depuis ladite chambre de pompe (216) étant défini entre ladite tige de pompe (238) et l'intérieur de ladite structure tubulaire (214) lors du déplacement de ladite tige de pompe (238) vers ladite position avancée,
- ledit conduit d'alimentation en dose (235) reliant ledit passage d'écoulement allongé (230) à ladite chambre de retenue de liquide (92) et incluant un clapet anti-retour (207, 208) permettant l'écoulement dudit liquide vers ladite chambre de retenue de liquide (92).

2. Dispositif (1) selon la revendication 1, ladite seconde partie (225) présentant une paroi périphérique (229) entourant ladite première partie (210) dans ladite position avancée de ladite tige de pompe (238).

3. Dispositif (1) selon la revendication 1 ou 2, incluant une chambre supplémentaire (21) communiquant avec ladite chambre de dose unique (92) par l'intermédiaire de ladite ouverture de décharge (93), et un conduit d'écoulement d'air supplémentaire (78) pour fournir un second écoulement d'air à ladite chambre supplémentaire (21), ladite chambre supplémentaire (21) présentant une ouverture de décharge (20) pour la décharge depuis ledit dispositif (1) de ladite dose unique expulsée de force, conjointement avec ledit second écoulement d'air.

4. Dispositif selon l'une quelconque des revendications précédentes, comprenant en outre un ressort (150) entourant ladite première partie (210), pour commander ledit déplacement de ladite tige de pompe (238) à l'intérieur de ladite chambre de pompe (216) entre lesdites positions rétractée et avancée.

5. Dispositif selon l'une quelconque des revendications précédentes, ledit conduit d'alimentation en dose (235) présentant un orifice d'entrée de liquide (EP) agencé au niveau d'une extrémité proximale de ladite structure tubulaire (214), de préférence adjacent à ladite ouverture (217), un segment (235‴) dudit conduit d'alimentation en dose (235) s'étendant de préférence latéralement par rapport à ladite tige de pompe (238).

6. Dispositif (1) selon l'une quelconque des revendications précédentes, dans lequel ledit contenant (70) est déformable.

7. Dispositif (1) selon l'une quelconque des revendications précédentes, incluant une chambre à air (58) reliée audit conduit d'écoulement d'air (76, 77), un piston déplaçable (55) pour expulser un volume d'air hors de ladite chambre à air (58), et un mécanisme d'entraînement pour commander le déplacement dudit piston (55).

8. Dispositif (1) selon la revendication précédente, comprenant en outre une poignée rotative (8), ledit piston (55) incluant une tige de piston (57) et une tête de piston (56) reçues dans ladite chambre à air (58), dans lequel la rotation de ladite poignée (8) déplace ledit piston (55) vers une position rétractée pour aspirer ledit volume d'air jusque dans ladite chambre à air (58), et dans lequel ladite rotation de ladite poignée (8) commande ledit déplacement de ladite seconde partie (225) par rapport à ladite première partie (210).

9. Dispositif selon l'une quelconque des revendications précédentes, ladite première partie (210) incluant ledit conduit d'alimentation en dose (235).
